(19) Europäisches Patentamt / European Patent Office / Office européen des brevets

(11) **EP 4 670 730 A1**

(12) **EUROPEAN PATENT APPLICATION**

(43) Date of publication:
**31.12.2025 Bulletin 2026/01**

(21) Application number: **24223872.3**

(22) Date of filing: **31.12.2024**

(51) International Patent Classification (IPC):
*A61K 36/428* (2006.01)  *A61K 38/01* (2006.01)
*A61K 38/04* (2006.01)  *A61P 5/26* (2006.01)
*A61P 13/12* (2006.01)  *C07K 7/00* (2006.01)
*C12P 21/06* (2006.01)  *A01H 5/10* (2018.01)
*A01H 6/34* (2018.01)  *C07K 14/415* (2006.01)

(52) Cooperative Patent Classification (CPC):
A61K 36/428; A01H 5/10; A01H 6/34;
A61K 38/011; A61K 38/04; A61P 5/26;
A61P 13/12; C07K 14/415; C12P 21/06;
A61K 2236/00

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(30) Priority: **25.06.2024 CN 202410828562**

(71) Applicants:
• **South China Agricultural University**
**Guangzhou, Guangdong 510642 (CN)**
• **Fung-wong Pharmaceutical Group (Zhuhai) Co.,
Ltd**
**Zhuhai, Guangdong 519099 (CN)**

(72) Inventors:
• **DU, Bing**
**Guangzhou, 510642 (CN)**

• **HE, Zhilin**
**Guangzhou, 510642 (CN)**
• **LI, Pan**
**Guangzhou, 510642 (CN)**
• **PENG, Dong**
**Guangzhou, 510642 (CN)**
• **ZENG, Jieyu**
**Guangzhou, 510642 (CN)**

(74) Representative: **Bayramoglu et al.**
**Mira Office
Kanuni Sultan Süleyman Boulevard 5387
Street Beytepe, floor 12, no:50
06800 Cankaya, Ankara (TR)**

Remarks:
•The complete document including Reference
Table(s) and the Sequence Listing(s) can be
downloaded from the EPO website
•Claims filed after the date of filing of the application
(Rule 68(4) EPC).

(54) **FRUCTUS TRICHOSANTHIS POLYPEPTIDE HAVING EFFICACY OF TONIFYING KIDNEY YANG, PREPARATION METHOD AND USE THEREOF**

(57) The present invention belongs to the field of biotechnology, and specifically relates to a *Fructus Trichosanthis* polypeptide having the efficacy of tonifying kidney yang, a preparation method and use thereof. The present invention prepares the *Fructus Trichosanthis* polypeptide having the efficacy of tonifying kidney and invigorating yang by processes such as pretreatment of a *Fructus Trichosanthis* raw material, preparation of a *Fructus Trichosanthis* seed solution, preparation of a crude *Fructus Trichosanthis* peptide, ion-exchange resin chromatography, and purification via high performance liquid chromatography. The *Fructus Trichosanthis* polypeptide provided by the present invention can modulate the expression of genes and proteins associated with secretion of testosterone in TM3 cells, enhance the function of mitochondria, accelerate the expression of genes and proteins associated with mitochondrial biogenesis, significantly promote the secretion of testosterone in TM3 cells, and has the efficacy of tonifying kidney and invigorating yang, thereby expanding the application range of *Fructus Trichosanthis*.

FIG. 7

EP 4 670 730 A1

## Description

### TECHNICAL FIELD

The present invention belongs to the field of biotechnology, and specifically relates to a *Fructus Trichosanthis* polypeptide having the efficacy of tonifying kidney yang, a preparation method and use thereof.

### BACKGROUND

Testosterone is a kind of important male hormone and plays a crucial role in modulating sexual functions, reproduction, muscular movement, and various physiological activities. The latest research shows that the shortage of testosterone is possibly correlated to diseases such as obesity, cardiovascular diseases and even depressive disorder. Each age group of males may suffer from testosterone deficiency. In adult male, such deficiency usually attributes to natural decline in the level of endogenous testosterone caused by aging and other changeable factors. Currently, exogenous testosterone replacement therapy (TRT) still has certain side effects. Therefore, it is of great significance to seek for a substance capable of accelerating the synthesis of endogenous testosterone.

There are relatively common traditional Chinese medicines for tonifying kidney and invigorating yang in clinical, such as sea horse, *herba epimedii, Semen Cuscutae,* and *Cistanche deserticola,* but these have certain side effects and thus are hard to guarantee safety of human body after being taken for a long time. *Fructus Trichosanthis* belongs to *Lagenaria* of Cucurbitaceae; it is a traditional Chinese oil-bearing crop and has been widely planted and popularized. *Fructus Trichosanthis* seed is rich in protein (26.70%) and has extremely high nutritive value; besides being rich in arginine, leucine, and glutamic acid, its amino acid composition is close to the content of essential amino acids recommended by the World Health Organization. There are a few of studies on the effect and active substances of the *Fructus Trichosanthis* seed at present; it needs to make a further study to expand the applications of *Fructus Trichosanthis.*

### SUMMARY

The present invention provides a *Fructus Trichosanthis* polypeptide having the efficacy of tonifying kidney and invigorating yang, a preparation method and use thereof. The preparation method provided by the present invention can obtain a *Fructus Trichosanthis* polypeptide having the efficacy of tonifying kidney and invigorating yang from *Fructus Trichosanthis,* and has a simple preparation process and a high polypeptide yield. The obtained *Fructus Trichosanthis* polypeptide is safe and small in molecule and thus, can be absorbed and utilized by human body better, and can significantly improve the testosterone secretion in TM3 cells, and can be developed as a substitute for a traditional invigorating-yang medicine.

In a first aspect, the present invention provides a preparation method of a *Fructus Trichosanthis* polypeptide, specifically including the following steps:

step 1: taking and degreasing a dehulled and crushed *Fructus Trichosanthis* seed to remove phenolic substances, and performing drying and pulverizing to obtain a *Fructus Trichosanthis* seed powder;

step 2: performing a heated water extracting to the *Fructus Trichosanthis* seed powder to obtain a *Fructus Trichosanthis* seed solution containing degenerated protein;

step 3: performing enzymatic hydrolysis by composite enzyme to the *Fructus Trichosanthis* seed solution; after the enzymatic hydrolysis, performing enzyme deactivation and solid-liquid separation, collecting an enzymatic hydrolysate, then drying to obtain a crude *Fructus Trichosanthis* peptide; where the composite enzyme comprises a neutral protease and an alkaline protease;

step 4: dissolving the crude *Fructus Trichosanthis* peptide into water to prepare a crude *Fructus Trichosanthis* peptide solution, filtering the crude *Fructus Trichosanthis* peptide solution, and performing ion-exchange resin chromatography on a filtrate obtained; serving double distilled water as an eluent, and collecting the eluent of a corresponding peak according to an absorbance curve, followed by drying to obtain an ion-exchange chromatography enzymolysis product, wherein a cellulose anion-exchange chromatographic column is used in the ion-exchange resin chromatography; and

step 5: dissolving the ion-exchange chromatography enzymolysis product into water, performing separation and purification by reversed-phase high-performance liquid chromatography (RP-HPLC), and collecting a protein polypeptide capable of significantly accelerating secretion of testosterone in a TM3 cell to obtain a *Fructus Trichosanthis* polypeptide; in the RP-HPLC, a chromatographic column is a C18 chromatographic column; a mobile phase A is a 0.1%-0.2% trifluoroacetic acid-aqueous solution; a mobile phase B is a 0. 1%-0.2% trifluoroacetic acid-acetonitrile solution; and gradient elution is performed by the following procedure:

for 0-3 min in a mobile phase of a 95% mobile phase A + a 5% mobile phase B;

for 3-10 min in a mobile phase of an 80% mobile phase A + a 20% mobile phase B;

for 10-20 min in a mobile phase of a 50% mobile phase A + a 50% mobile phase B; and

for 20-23 min in a mobile phase of a 20% mobile phase A + an 80% mobile phase B.

*Fructus Trichosanthis* seed is rich in protein; after protein is subjected to enzymolysis, there are more by-products in the enzymatic hydrolysate, i.e., mixed peptides; there exist great difficulties in the screening of a target polypeptide. Moreover, as an oil crop, *Fructus Trichosanthis* is rich in grease and polyphenol substances; grease causes a strong interference effect on the protein-purified column, and the polyphenol substances will bind a protein to hinder the obtaining of protein and reduce the extraction rate. In the preparation method of the *Fructus Trichosanthis* polypeptide provided by the present invention, *Fructus Trichosanthis* seed, as raw material, is degreased and subjected to phenol removal, and then cooked at high temperature to degenerate protein, and followed by enzymolysis, and then isolated and purified.at multi-stages. In each stage of isolation and purification, parameters of the eluent such as type, concentration, and flow rate are controlled, to prepare the *Fructus Trichosanthis* polypeptide having the efficacy of tonifying kidney and invigorating yang finally. The method can accurately determine the elution peak of the target polypeptide by detecting the absorbance curve, and can collect the target polypeptide repeatedly and stably by controlling the time to collecting the eluent.

In step 1, the *Fructus Trichosanthis* seed is dried and powdered to facilitate the extraction of protein, and the dried *Fructus Trichosanthis* seed is crushed to a grain size of not greater than 0.5 mm.

In step 3, the solid-liquid separation is performed by filtering or centrifugation, and the corresponding filtrate or supernatant is collected to obtain the enzymatic hydrolysate. In steps 3 and 4, the drying way may be direct drying, drying after concentration, or drying after distillation under reduced pressure.

In step 4, double distilled water serves as an eluent; the ion-exchange chromatography enzymolysis product has the maximum yield.

In step 5, significantly accelerating secretion of testosterone in a TM3 cell refers that the effect of the screened polypeptide in improving secretion of testosterone in a TM3 cell has a statistically significant difference as compared to the secretion of original testosterone in a TM3 cell.

Preferably, in the step 1, a degreasing solvent for the *Fructus Trichosanthis* seed is n-hexane; a weight-to-volume ratio of the *Fructus Trichosanthis* seed to the n-hexane is 1:3-6; a solvent for removing phenolic substances is acetone; a weight-to-volume ratio of the *Fructus Trichosanthis* seed to the acetone is 1:3-6.

Preferably, in the step 2, the heating and water-extracting is performed at a temperature of 90-100°C for 3-10 h, and a mass ratio of the *Fructus Trichosanthis* seed powder to water is 1 :30-50.

The high temperature of 90-100°C is beneficial to the subsequent enzymolysis.

Preferably, in the step 3, the neutral protease is a metalloprotease derived from *Bacillus subtilis.*

*Bacillus subtilis-derived* neutral protease is a kind of metalloprotease, and it is a kind of endo protease extracted from *Bacillus subtilis* via submerged fermentation cultivation; it is a pure-natural, safe and non-toxic, and has strong hydrolysis ability, and can decompose macromolecular protein into polypeptides, amino acids, and other products.

Preferably, the alkaline protease is an endo protease derived from *Bacillus licheniformis.*

*Bacillus licheniformis-derived* alkaline protease is an enzymic preparation fermented from *Bacillus licheniformis* via submerged liquid fermentation; it can rapidly decompose proteins and hydrolyze macromolecular protein into free amino acids, and other products.

More preferably, an amount of the alkaline protease added accounts for 20%-40% of the *Fructus Trichosanthis* seed powder by mass; an amount of the neutral protease accounts for 20%-40% of the *Fructus Trichosanthis* seed powder by mass; in the step 3, the composite enzymatic hydrolysis is performed at a pH of 7.5-8 or 9-9.5 and a temperature of 55-75°C for 3-5 h.

More preferably, in the step 3, the composite enzymatic hydrolysis is performed at a pH of 9-9.5 and a temperature of 65°C for 5 h. The yield of the crude *Fructus Trichosanthis* peptide is up to the maximum under such an enzymolysis process.

Preferably, in the step 4, the eluent has a flow rate of 1-2 mL/min; a DEAE-52 cellulose anion-exchange chromatographic column is used in the ion-exchange resin chromatography.

More preferably, in the step 4, the eluent has a flow rate of 1 mL/min, and an eluent having an appearance time of 135-250 min is collected.

The appearance time of the ion-exchange chromatography enzymolysis product is 135-250 min at such a flow rate.

Preferably, in the step 5, a chromatographic column is Pursuit XRs C-18; the mobile phase A and the mobile phase B are 0.1% trifluoroacetic acid-water and 0.1% trifluoroacetic acid-acetonitrile, respectively, and have a flow rate of 20 mL/min; an eluent having an appearance time of 10.0-12.3 min or 21.0-21.4 min is collected, to obtain a *Fructus Trichosanthis* polypeptide.

The *Fructus Trichosanthis* polypeptides obtained from the eluent having the appearance time of 10.0-12.3 min and from the eluent having the appearance time of 21.0-21.4 min all have significant acceleration to the secretion of testosterone in a TM3 cell.

In a second aspect, the present invention provides a *Fructus Trichosanthis* polypeptide prepared by the above method.

The *Fructus Trichosanthis* polypeptide provided by the present invention mainly includes small molecule peptides, is safe to human body and good in absorption, can suppress or accelerate the secretion of testosterone in a TM3 cell, promote the mitochondrial biogenesis, and has the efficacy of tonifying kidney and invigorating yang. The mechanism of the *Fructus Trichosanthis* polypeptide in accelerating the secretion of testosterone in a TM3 cell is correlated to the expression of the related genes and proteins for modulating the secretion of testosterone as well as the expression of genes and proteins for accelerating mitochondrial biogenesis.

In a third aspect, the present invention provides use of the above *Fructus Trichosanthis* polypeptide, including for use in manufacture of a medicament for tonifying kidney and invigorating yang; or

for use in a medicament for accelerating a TM3 cell to secrete testosterone, or
for use in manufacture of a medicament for modulating expression of a gene and a protein associated with secretion of testosterone in a TM3 cell; or
for use in manufacture of a medicament for modulating expression of a gene and a protein associated with mitochondrial biogenesis in a TM3 cell.

**BRIEF DESCRIPTION OF THE DRAWINGS**

To specify the examples of the present invention or the technical solutions in the prior art more clearly, the accompanying drawings described in the examples or prior art will be introduced briefly hereafter. Obviously, the accompanying drawings described below are merely some embodiments of the present invention. Those skilled in the art may further obtain other drawings without any inventive efforts.

FIG. 1 shows an absorbance curve of an ion-exchange chromatography enzymolysis product obtained after elution with different eluents in the present invention;
FIG. 2 shows an absorbance curve of a *Fructus Trichosanthis* polypeptide purified by RP-HPLC;
FIG. 3 shows a total ion current chromatogram of a *Fructus Trichosanthis* polypeptide TSH1-3 obtained in Example 2 of the present invention;
FIG. 4 shows screening results of a crude *Fructus Trichosanthis* peptide via a water-extracting process and enzymolysis process in Example 1 of the present invention;
FIG. 5 shows an impact of the *Fructus Trichosanthis* polypeptide on survival rate of a TM3 cell according to the present invention;
FIG. 6 shows an impact of the ion-exchange chromatography enzymolysis product on testosterone secretion in a TM3 cell in the present invention;
FIG. 7 shows an impact of the *Fructus Trichosanthis* polypeptide on testosterone secretion in a TM3 cell according to the present invention;
FIG. 8 shows impacts of the *Fructus Trichosanthis* polypeptide TSH1-3 and a synthetic peptide fragment VTPVGSPR on the secretion volume of a TM3 cell-correlated male hormone as well as the levels of ATP and mitochondrial membrane potential in the present invention;
FIG. 9 shows impacts of the *Fructus Trichosanthis* polypeptide TSH1-3 and the synthetic peptide fragment VTPVGSPR on the expression of genes correlated to synthesis of testosterone in the present invention;
FIG. 10 shows impacts of the *Fructus Trichosanthis* polypeptide TSH1-3 and the synthetic peptide fragment VTPVGSPR on the expression of proteins correlated to synthesis of testosterone in the present invention;
FIG. 11 shows impacts of the *Fructus Trichosanthis* polypeptide TSH1-3 and the synthetic peptide fragment VTPVGSPR on the replication of mitochondrial mtDNA in the present invention;
FIG. 12 shows impacts of the *Fructus Trichosanthis* polypeptide TSH1-3 and the synthetic peptide fragment VTPVGSPR on the expression of proteins correlated to mitochondrial biogenesis in the present invention; and
FIG. 13 shows impacts of the *Fructus Trichosanthis* polypeptide TSH1-3 and the synthetic peptide fragment VTPVGSPR on the expression of genes correlated to mitochondrial biogenesis in the present invention.

**DETAILED DESCRIPTION OF EMBODIMENTS**

The technical solutions in the embodiments of the present invention will be described clearly and completely in combination with the embodiments of the present invention. Obviously, the described examples are merely a part of embodiments of the present invention, but are not all the embodiments. Based on the embodiments of the present invention, any other embodiments obtained by those skilled in the art without any inventive efforts shall all fall within the protection scope of the present invention.

(I) Reagents: n-hexane, Macklin H810749; acetone, Guangzhou Chemical Reagent Factory HB02; trifluoroacetic acid (TFA), Macklin T818778; acetonitrile, Macklin A800362; ion exchange resin, DEAE-52, Solarbio C8930; penicillin-streptomycin solution (100X), Beyotime, C0222; CCK8 detection reagent, Abbkine KTA1020; BCA protein quantification kit, Abiowell AWB0104; RIPAlysate (strong) Abiowell AWB0136; pancreatin gibco 25200056; ECL Plus hypersensitive luminescent solution Abiowell AWB0005; alkaline protease alcalase 2.4L, Novozymes; neutral protease Nuetrase 0.8L, Novozymes; mouse testosterone (T)ELISA scientific research kit, MM-0569M1, Jiangsu Meimian Industrial Co., Ltd; superoxide dismutase (SOD) kit G0101W, Suzhou Grace Biotechnolgy Co., Ltd.; malondialdehyde (MDA) kit, G0109W, Suzhou Grace Biotechnolgy Co., Ltd.

(II) Equipment: the chromatographic column Pursuit XRs C-18 of the reversed-phase high-performance liquid chromatograph was purchased from Agilent Technology.

**Example 1**

The example provides a *Fructus Trichosanthis* polypeptide.

The specific preparation method is as follows:

Step 1: *Fructus Trichosanthis* seed was dehulled and crushed. N-hexane was added in a solid-liquid ratio of 1:5 (g:mL) for degreasing, and acetone was added in a solid-liquid ratio of 1:5 (g:mL) to remove phenolic substances, the obtained product was dried in air and sieved with an 80-meshed sieve to obtain a *Fructus Trichosanthis* seed powder.

Step 2: 10 g of the *Fructus Trichosanthis* seed powder were taken and added with purified water and mixed in a solid-liquid ratio of 1:40 (g:mL), and then heated in a 95°C water bath, and thermally insulated for 5 h to obtain a *Fructus Trichosanthis* seed solution.

Step 3: the *Fructus Trichosanthis* seed solution was cooled to 65°C, and added with 2 mL alkaline protease and 2 mL neutral protease for enzymolysis at a pH of 9 and 65°C for 5 h, and then subjected to enzyme deactivation at 95°C for 15 min at the end of the enzymolysis, afterwards centrifuged to collect solution, the collected solution was subjected to distillation under reduced pressure and frozen-dried to obtain a crude *Fructus Trichosanthis* peptide.

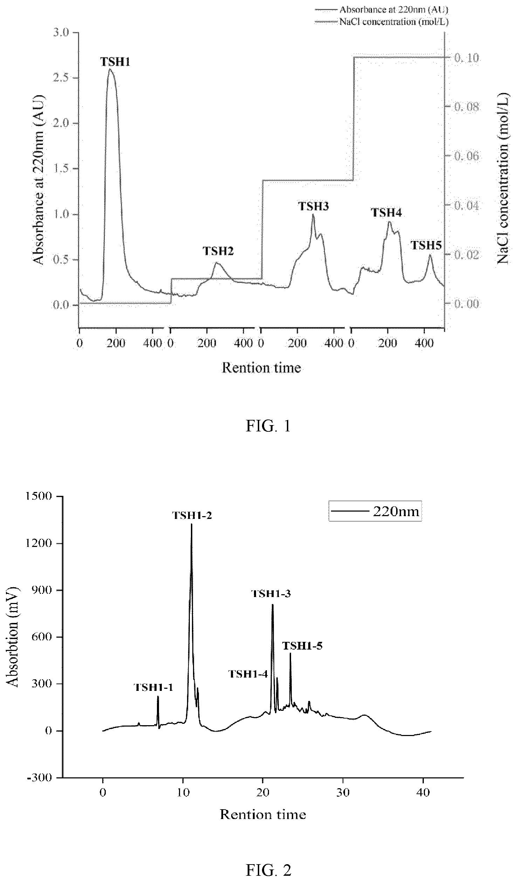

Step 4: the crude *Fructus Trichosanthis* peptide was dissolved and diluted to 20 mg/ml, and then filtered with a 0.22 μm filter membrane, and sampled by a pressure pump to enter into a DEAE-52 cellulose anion-exchange chromatographic column, and eluted by double distilled water at a flow rate of 1 mL/min; the absorbance curve of the eluent at 220 nm is shown in FIG. 1; components having elution time of 135-250 min were collected, concentrated under reduced pressure and frozen-dried in vacuum to obtain an ion-exchange chromatography enzymolysis product TSH1.

Step 5: TSH1 was dissolved into double distilled water and prepared to 5 mg/mL, then filtered with a 0.22 μm filter membrane, isolated and purified by RP-HPLC; the chromatographic column was Pursuit XRs C-18 at a column temperature of 35°C; the sampling volume was 600 μL; the mobile phase A was 0.1% TFA-water, and the mobile phase B was 0.1% TFA-acetonitrile; the mobile phases had a flow rate of 20 mL/min. The sequence of gradient elution is specifically shown in the table below:

Table 1 Sequence of linear gradient elution of the mobile phase

| Time (min) | A (0.1% TFA-water) | B (0.1% TFA-acetonitrile) |
|---|---|---|
| 0-3 | 95% | 5% |
| 3-10 | 80% | 20% |
| 10-20 | 50% | 50% |
| 20-23 | 20% | 80% |
| 23-25 | 95% | 5% |

Absorbance curves of the eluent at 220 nm in different periods of time are shown in FIG. 2. The eluent having elution time of 21.0-21.4 min was collected, concentrated under reduced pressure, and frozen-dried in vacuum to obtain a *Fructus Trichosanthis* polypeptide SH1-3.

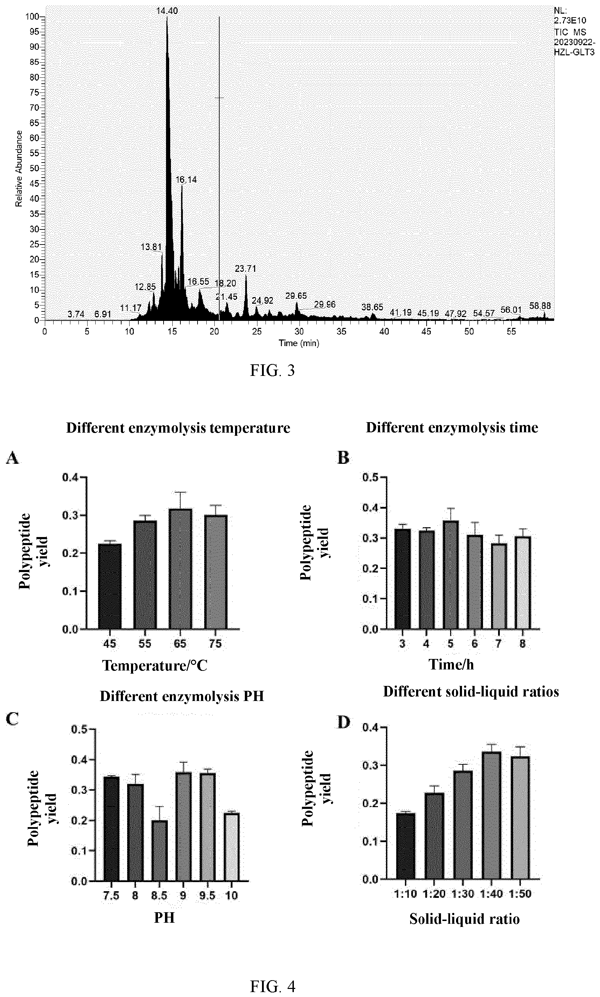

The component TSH1-3 was subjected to peptide fragment identification by LC-MS/MS and high-resolution mass spectral database set, and its amino acid sequence was analyzed, to finally obtain 42 peptide fragments having a higher confidence level; their total ion current diagrams are shown in FIG. 3; the amino acid sequences of the 42 peptide fragments are shown in Table 2.

Table 2 Composition of peptide fragments of the TSH 1-3

| Polypeptide sequence | SE Q ID NO: | Confidence level | Mass number | Length | Mass-to-charge ratio |
|---|---|---|---|---|---|
| NAHSATTWSGQYVGGAEAR | 2 | 67.32 | 1961.898 | 19 | 654.9739 |
| HTACPLP | 3 | 47.37 | 737.353 | 7 | 738.3641 |
| EGRQLLDKARNLPS | 4 | 45.64 | 1595.874 | 14 | 532.9662 |
| GRQLLDKARNLPS | 5 | 44.59 | 1466.832 | 13 | 489.9511 |
| LGEYGFQNALIVR | 6 | 42.27 | 1478.788 | 13 | 740.4017 |
| QRVLSP | 7 | 41.9 | 681.381 | 6 | 682.3888 |
| SATTWSGQYVGGAEAR | 8 | 41.14 | 1639.759 | 16 | 820.8914 |
| LLDKARNLPS | 9 | 40.36 | 1125.651 | 10 | 563.8323 |
| VLDPNTVFALVNYISFK | 10 | 37.84 | 1939.045 | 17 | 647.3563 |
| PSIKPP | 11 | 36.51 | 637.3799 | 6 | 638.3885 |
| NAHSATTWSGQYVGGAEAR | 12 | 33.9 | 1962.882 | 19 | 655.3012 |
| GEELFTGVVPILVELDGDVN GHK | 13 | 33.14 | 2436.254 | 23 | 813.0919 |
| PCHCR | 14 | 32.31 | 614.2417 | 5 | 615.2488 |
| LIPQQ | 15 | 31.52 | 597.3486 | 5 | 598.3556 |
| NVKVA | 16 | 31.24 | 529.3224 | 5 | 530.3293 |
| GVAAIAGV | 17 | 30.55 | 656.3857 | 8 | 657.393 |
| PRTPAGP | 18 | 30.49 | 694.3762 | 7 | 695.3838 |
| VLPVPQ | 19 | 30.27 | 651.3955 | 6 | 652.4028 |
| PPTRPGA | 20 | 30 | 694.3762 | 7 | 695.3831 |
| VTPVGSPR | 1 | 29.87 | 811.4552 | 8 | 406.7364 |
| DPGPRGER | 21 | 28.83 | 882.4307 | 8 | 442.2224 |
| SGSTSK | 22 | 28.59 | 565.2708 | 6 | 566.2817 |
| DAFLGSFLYEYSR | 23 | 28.03 | 1566.735 | 13 | 784.375 |
| TPQPGV | 24 | 27.96 | 597.3122 | 6 | 598.3175 |
| RGVGLGPR | 25 | 27.92 | 810.4824 | 8 | 406.2487 |
| GGGRFGGG | 26 | 27.84 | 663.3088 | 8 | 664.3209 |
| RQHNNIPLETPQKKFFEGEM R | 27 | 27.83 | 2600.281 | 21 | 651.0755 |
| NVLSP | 28 | 27.81 | 528.2908 | 5 | 529.2979 |
| VLVPSQ | 29 | 27.49 | 641.3748 | 6 | 642.3815 |
| SPDLPK | 30 | 27.44 | 655.3541 | 6 | 656.3638 |
| CDIPPR | 31 | 27.32 | 699.3374 | 6 | 700.3443 |
| SVTTSP | 32 | 27.29 | 590.2911 | 6 | 591.2952 |
| TPAPR | 33 | 27.23 | 540.302 | 5 | 541.3094 |

(continued)

| Polypeptide sequence | SE Q ID NO: | Confidence level | Mass number | Length | Mass-to-charge ratio |
|---|---|---|---|---|---|
| VNLPS | 34 | 26.76 | 528.2908 | 5 | 529.2982 |
| EPGPQ | 35 | 26.59 | 526.2387 | 5 | 527.2423 |
| ESGAPGV | 36 | 26.36 | 597.2758 | 7 | 598.2835 |
| VQDPAPR | 37 | 25.98 | 781.4082 | 7 | 782.4163 |
| SGSCGR | 38 | 25.93 | 565.2278 | 6 | 566.2303 |
| RVPVPS | 39 | 25.86 | 653.386 | 6 | 654.3926 |
| EGRQLLDKAR | 40 | 25.85 | 1184.663 | 10 | 593.3383 |
| GVPGIAGPR | 41 | 25.17 | 822.4711 | 9 | 412.246 |
| HDTFT | 42 | 25.16 | 619.2602 | 5 | 620.265 |

**Comparative Examples 1-4**

Comparative Examples 1-4 provide three ion-exchange chromatography enzymolysis products TSH2, TSH3, TSH4, and TSH5, respectively.

The crude *Fructus Trichosanthis* peptide prepared in step 3 of Example 1 was taken and subjected to ion-exchange resin chromatography according to step 4 of Example 1; it differs from step 4 of Example 1 in that double distilled water was replaced by sodium chloride having a concentration of 0.01 mol/L for elution, and components having elution time of 168-264 min were collected, concentrated under reduced pressure and frozen-dried in vacuum to obtain an ion-exchange chromatography enzymolysis product TSH2.

The crude *Fructus Trichosanthis* peptide prepared in step 3 of Example 1 was taken and subjected to ion-exchange resin chromatography according to step 4 of Example 1; it differs from step 4 of Example 1 in that double distilled water was replaced by sodium chloride having a concentration of 0.05 mol/L for elution, and components having elution time of 116-292 min were collected, concentrated under reduced pressure and frozen-dried in vacuum to obtain an ion-exchange chromatography enzymolysis product TSH3.

The crude *Fructus Trichosanthis* peptide prepared in step 3 of Example 1 was taken and subjected to ion-exchange resin chromatography according to step 4 of Example 1; it differs from step 4 of Example 1 in that double distilled water was replaced by sodium chloride having a concentration of 0.1mol/L for elution, and components having elution time of 116-212 min were collected, concentrated under reduced pressure and frozen-dried in vacuum to obtain an ion-exchange chromatography enzymolysis product TSH4; components having elution time of 308-368 min were collected, concentrated under reduced pressure and frozen-dried in vacuum to obtain an ion-exchange chromatography enzymolysis product TSH5.

The absorbance curves of the ion-exchange chromatography enzymolysis products in Comparative Examples 1-4 are shown in FIG. 1.

**Example 2**

The Example 2 provides a *Fructus Trichosanthis* polypeptide TSH1-2.

The ion-exchange chromatography enzymolysis product TSH1 prepared in step 4 of Example 1 was taken, isolated and purified by RP-HPLC according to step 5 of Example 1; it differs from step 5 of Example 1 in that the collection time of the TSH1-2 eluent was 10.0-12.3 min.

The absorbance curve of the *Fructus Trichosanthis* polypeptide in Example 2 is shown in FIG. 2.

**Comparative Examples 5-7**

Comparative Examples 5-7 provide *Fructus Trichosanthis* polypeptides TSH1-1, TSH1-4, and TSH1-5.

The ion-exchange chromatography enzymolysis product TSH1 prepared in step 4 of Example 1 was taken, isolated and purified by RP-HPLC according to step 5 of Example 1; it differs from step 5 of Example 1 in that the collection time of the eluents for TSH1-1, TSH1-4, and TSH1-5 was 6.7-7.0 min, 21.6-22.0 min, and 23.2-23.6 min, respectively.

The absorbance curves of the *Fructus Trichosanthis* polypeptides in Comparative Examples 5-7 are shown in FIG. 2.

### Example 3

Example 3 provides a *Fructus Trichosanthis* polypeptide.

Step 1: *Fructus Trichosanthis* seed was dehulled and crushed. N-hexane was added in a solid-liquid ratio of 1:6 (g:mL) for degreasing, and acetone was added in a solid-liquid ratio of 1:6 (g:mL) to remove phenolic substances, the obtained product was dried in air and sieved with an 80-meshed sieve to obtain a *Fructus Trichosanthis* seed powder.

Step 2: 10 g of the *Fructus Trichosanthis* seed powder were taken and added with purified water and mixed in a solid-liquid ratio of 1:5 (g:mL), and then heated in a 100°C water bath, and thermally insulated for 10 h to obtain a *Fructus Trichosanthis* seed solution.

Step 3: the *Fructus Trichosanthis* seed solution was cooled to 50°C, and added with 4 mL alkaline protease and 4 mL neutral protease for enzymolysis at a pH of 7.5 and 55°C for 5 h, and then subjected to enzyme deactivation at 95°C for 15 min at the end of the enzymolysis, afterwards centrifuged to collect solution, the collected solution was subjected to distillation under reduced pressure and frozen-dried to obtain a crude *Fructus Trichosanthis* peptide.

The remaining steps are the same as those in Example 1 to obtain a *Fructus Trichosanthis* polypeptide.

### Example 4

Example 4 provides a *Fructus Trichosanthis* polypeptide.

Step 1: *Fructus Trichosanthis* seed was dehulled and crushed. N-hexane was added in a solid-liquid ratio of 1:3 (g:mL) for degreasing, and acetone was added in a solid-liquid ratio of 1:3 (g:mL) to remove phenolic substances, the obtained product was dried in air and sieved with an 80-meshed sieve to obtain a *Fructus Trichosanthis* seed powder.

Step 2: 10 g of the *Fructus Trichosanthis* seed powder were taken and added with purified water and mixed in a solid-liquid ratio of 1:50 AM (g:mL), and then heated in a 90°C water bath, and thermally insulated for 3 h to obtain a *Fructus Trichosanthis* seed solution.

Step 3: the *Fructus Trichosanthis* seed solution was cooled to 45°C, and added with 3 mL alkaline protease and 3 mL neutral protease for enzymolysis at a pH of 9.5 and 75°C for 3 h, and then subjected to enzyme deactivation at 95°C for 15 min at the end of the enzymolysis, afterwards centrifuged to collect solution, the collected solution was subjected to distillation under reduced pressure and frozen-dried to obtain a crude *Fructus Trichosanthis* peptide.

The remaining steps are the same as those in Example 1 to obtain a *Fructus Trichosanthis* polypeptide.

### Example 5

The example provides a crude *Fructus Trichosanthis* peptide.

Step 1: *Fructus Trichosanthis* seed was dehulled and crushed. N-hexane was added in a solid-liquid ratio of 1:5 (g:mL) for degreasing, and acetone was added in a solid-liquid ratio of 1:5 (g:mL) to remove phenolic substances, the obtained product was dried in air and sieved with a 80-meshed sieve to obtain a *Fructus Trichosanthis* seed powder.

Step 2: 10 g of the *Fructus Trichosanthis* seed powder were taken and added with purified water and mixed in a solid-liquid ratio of 1:40 (g:mL), and then heated in a 95°C water bath, and thermally insulated for 5 h to obtain a *Fructus Trichosanthis* seed solution.

Step 3: the *Fructus Trichosanthis* seed solution was cooled to enzymolysis temperature, and added with 2 mL alkaline protease and 2 mL neutral protease for enzymolysis at a pH of 9 and 75°C for 4 h; and then subjected to enzyme deactivation at 95°C for 15 min at the end of the enzymolysis, afterwards centrifuged to collect solution, the collected solution was subjected to distillation under reduced pressure and frozen-dried to obtain a crude *Fructus Trichosanthis* peptide.

### Example 6

Example 6 differs from Example 5 in that the enzymolysis temperature in step 3 is 65°C.

### Example 7

Example 7 differs from Example 5 in that the enzymolysis temperature in step 3 is 55°C.

### Comparative Example 8

Comparative Example 8 differs from Example 5 in that the enzymolysis temperature in step 3 is 45°C.

The yield of the crude *Fructus Trichosanthis* peptide at different enzymolysis temperature was calculated. The yield is calculated by the following formula:

$$Polypeptide\ yield = \frac{Polypeptide\ content * Liquid\ volume}{Solid\ weight\ before\ enzymolysis * Protein\ content}$$

Protein content: through Kjeldahl method for nitrogen determination, the protein content in the degreased *Fructus Trichosanthis* powder was 73.21%

Polypeptide content: the polypeptide content in the enzymatic hydrolysate was determined by a biuret method.

As shown in FIG. 1A, the results of Examples 5-7 and Comparative Example 8, enzymolysis temperature may comparatively greatly impact the yield of the polypeptide; the yield of the polypeptide is higher at enzymolysis temperature of 55-75°C, and the effect is up to the optimal at 65°C.

## Example 8

The example provides a crude *Fructus Trichosanthis* peptide.

The *Fructus Trichosanthis* seed solution prepared in step 2 of Example 5 was taken, cooled to 65°C, and added with 2 mL alkaline protease and 2 mL neutral protease for enzymolysis at a pH of 9 and 65°C for 3 h, and then subjected to enzyme deactivation at 95°C for 15 min at the end of the enzymolysis, afterwards, centrifuged to collect solution, the collected solution was subjected to distillation under reduced pressure and frozen-dried to obtain a crude *Fructus Trichosanthis* peptide.

## Example 9

Example 9 differs from Example 8 in that the enzymolysis time in step 3 is 4 h.

## Example 10

Example 10 differs from Example 8 in that the enzymolysis time in step 3 is 5 h.

## Comparative Example 9

Comparative Example 9 differs from Example 8 in that the enzymolysis time in step 3 is 6 h.

## Comparative Example 10

Comparative Example 10 differs from Example 8 in that the enzymolysis time in step 3 is 7 h.

## Comparative Example 11

Comparative Example 11 differs from Example 8 in that the enzymolysis time in step 3 is 8 h.

The yields of the crude *Fructus Trichosanthis* peptides in Examples 8-10 and Comparative Examples 9-11 were calculated. The results are shown in FIG. 1B; as such, the crude *Fructus Trichosanthis* after enzymolysis for 3-5 h has a higher yield, and the effect is up to the optimal at enzymolysis time of 5 h.

## Example 11

The example provides a crude *Fructus Trichosanthis* peptide.

The *Fructus Trichosanthis* seed solution prepared in step 2 of Example 5 was taken, cooled to 65°C, and added with 2 mL alkaline protease and 2 mL neutral protease for enzymolysis at a pH of 7.5 and 65°C for 5 h, and then subjected to enzyme deactivation at 95°C for 15 min at the end of the enzymolysis, afterwards centrifuged to collect solution, the collected solution was subjected to distillation under reduced pressure and frozen-dried to obtain a crude *Fructus Trichosanthis* peptide.

## Example 12

Example 12 differs from Example 11 in that the enzymolysis pH in step 3 is 8.

**Example 13**

Example 13 differs from Example 11 in that the enzymolysis pH in step 3 is 9.

**Example 14**

Example 14 differs from Example 11 in that the enzymolysis pH in step 3 is 9.5.

**Comparative Example 12**

Comparative Example 12 differs from Example 11 in that the enzymolysis pH in step 3 is 8.5.

**Comparative Example 13**

Comparative Example 13 differs from Example 11 in that the enzymolysis pH in step 3 is 10.
The yields of the crude *Fructus Trichosanthis* peptides in Examples 11-14 and Comparative Examples 12-13 were calculated. The results are shown in FIG. 1C; as such, the crude *Fructus Trichosanthis* after enzymolysis for 7.5-8 h or 9-9.5 has a higher yield, and the effect is up to the optimal at pH = 9.

**Example 15**

The example provides a crude *Fructus Trichosanthis* peptide.
Step 1: *Fructus Trichosanthis* seed was dehulled and crushed. N-hexane was added in a solid-liquid ratio of 1:5 (g:mL) for degreasing, and acetone was added in a solid-liquid ratio of 1:5 (g:mL) to remove phenolic substances, the obtained product was dried in air and sieved with a 80-meshed sieve to obtain a *Fructus Trichosanthis* seed powder.
Step 2: 10 g of the *Fructus Trichosanthis* seed powder were taken and added with purified water and mixed in a solid-liquid ratio of 1:30 AM (g:mL), and then heated in a 95°C water bath, and thermally insulated for 5 h to obtain a *Fructus Trichosanthis* seed solution.
Step 3: the *Fructus Trichosanthis* seed solutions prepared in different solid-liquid ratios were cooled to 65°C, and added with 2 mL alkaline protease and 2 mL neutral protease for enzymolysis at a pH of 9 and 65°C for 5 h, and then subjected to enzyme deactivation at 95°C for 15 min at the end of the enzymolysis, afterwards centrifuged to collect solution, the collected solution was subjected to distillation under reduced pressure and frozen-dried to obtain a crude *Fructus Trichosanthis* peptide.

**Example 16**

Example 16 differs from Example 15 in that the solid-liquid ratio in step 2 is 1:40.

**Example 17**

Example 17 differs from Example 15 in that the solid-liquid ratio in step 2 is 1:50.

**Comparative Example 14**

Comparative Example 14 differs from Example 15 in that the solid-liquid ratio in step 2 is 1:10.

**Comparative Example 15**

Comparative Example 15 differs from Example 15 in that the solid-liquid ratio in step 2 is 1:20.
The yields of the crude *Fructus Trichosanthis* peptides in Examples 15-17 and Comparative Examples 14-15 were calculated. The results are shown in FIG. 1D; as such, the crude *Fructus Trichosanthis* in a solid-liquid ratio of 1:30-50 has a higher yield, and the effect is up to the optimal at 1:40.

**Test Example 1** Evaluation on the efficacy of the *Fructus Trichosanthis* polypeptide in accelerating testosterone secretion

1. Cell culture

TM3 cells were cultured in a DMEM medium rich in 2.5% (v/v) fetal calf serum (FBS) and 5% horse serum (HS) and cultured at 37°C and 5% $CO_2$ with the addition of 1% penicillin-streptomycin solution until the logarithmic phase.

2. Impact of the ion-exchange chromatography enzymolysis product and *Fructus Trichosanthis* polypeptide on the survival rate of TM3 cells

The survival rate of TM3 cells was measured by a CCK8 method. The TM3 cell suspension in the logarithmic phase was taken and paved in a 96-well plate, 100 μL per well having a cell density of $10^5$ CFU/mL, and cultured for 24 h in a 5% CO2, 37°C incubator. After cell adherence, supernatant in the well was removed carefully, and then 100 uL of DMEM culture solution was added; the sample group (TSH1, TSH1-1, TSH1-2, TSH1-3, TSH1-4, and TSH1-5) was respectively the DMEM culture solutions containing 200 μg/mL TSH1, 200 μg/mL TSH1-1, 200 μg/mL TSH1-2, 200 μg/mL TSH1-3, 200 μg/mL TSH1-4, and 200 μg/mL TSH1-5 (prepared in Examples 1, 2, and Comparative Examples 5-7, respectively); the DMEM culture solution in the control group and blank group contains no the above polypeptide. The 96-well plate was continuously put to an incubator for culture for 48 h, and then 10 μL CCK8 solution was added to the sample group and the control group, and equivalent weight of deionized water was added to the blank group, then the well plate was continuously incubated in the incubator for 4 h, and placed into a microplate reader to determine the absorbance at 450 nm, and then the cell survival rate was calculated. The cell survival rate is calculated by the following formula:

$$\text{Cell survival rate (\%)} = \frac{A0 - A1}{A0 - A2} \times 100\%;$$

where, A0 denotes an absorbance value of the sample group; A1 denotes an absorbance value of the control group, and A2 denotes an absorbance value of the blank group.

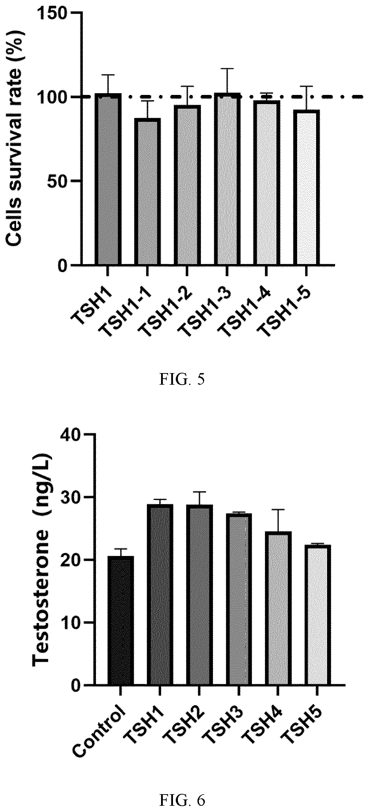

Impacts of the TSH1, TSH1-1, TSH1-2, TSH1-3, TSH1-4, and TSH1-5 on the survival rate of TM3 cells are shown in FIG. 5. As can be seen from FIG. 5, each component has no significant impact on the survival rate of the TM3 cell, indicating that the component has no significant toxicity to the TM3 cell.

3. Impact of the *Fructus Trichosanthis* polypeptide on testosterone secretion in a TM3 cell

The secretion of testosterone in a TM3 cell was measured by an Elisa kit. The TM3 cell suspension in the logarithmic phase was taken and paved in a 96-well plate, 100 μL per well having a cell density of $10^5$ CFU/mL, and cultured for 24 h in a 5% CO2, 37°C incubator. After cell adherence, supernatant in the well was removed carefully, and then 100 uL of DMEM culture solution was added; the sample group 1 (TSH1, TSH2, TSH3, TSH4, and TSH5) was respectively the DMEM culture solutions containing 200 μg/mL TSH1, 200 μg/mL TSH2, 200 μg/mL TSH3, 200 μg/mL TSH4, 200 μg/mL, and 200 μg/mL TSH5 (prepared in Example 1, and Comparative Examples 1-4, respectively); sample group 2 (TSH1, TSH1-1, TSH1-2, TSH1-3, TSH1-4, and TSH1-5) was respectively the DMEM culture solutions containing 200 μg/mL TSH1, 200 μg/mL TSH1-1, 200 μg/mL TSH1-2, 200 μg/mL TSH1-3, 200 μg/mL TSH1-4, and TSH1-5 (prepared in Example 1, Example 2, and Comparative Examples 5-7, respectively); the DMEM culture solution in the control group (Control) contains no the above polypeptide. After continuous culture for 48 h, cell supernatant was collected, and the amount of testosterone in the supernatant was measured by an Elisa kit. The results are shown in FIGS. 6-7.

FIG. 6 shows an impact of the ion-exchange chromatography enzymolysis product on testosterone secretion in a TM3 cell according to the present invention; as can be seen from FIG. 6, 5 components can improve the secretion of testosterone in the TM3 cell at the concentration of 200 μg/mL, where TSH1 and TSH2 are equivalent in effect; the component TSH1 was selected for further screening in combination with the yield.

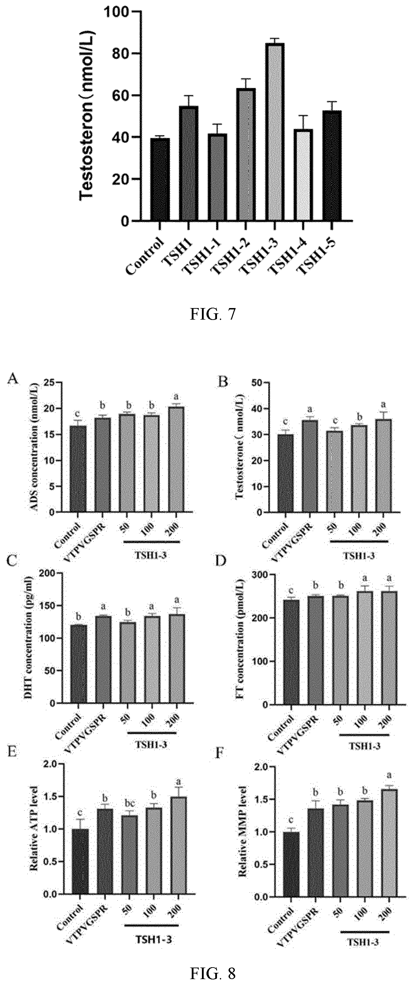

FIG. 7 shows an impact of the *Fructus Trichosanthis* polypeptide on testosterone secretion in a TM3 cell; as can be seen from FIG. 7 according to the present invention, both TSH1-2 and TSH1-3 may significantly improve the secretion of testosterone in the TM3 cell at the concentration of 200 μg/mL, while the effect of the TSH1-1, TSH1-4, and TSH1-5 similarly derived from TSH1 is inferior to the TSH1 in the secretion of testosterone in the TM3 cell at the concentration of 200 μg/mL. In the same bar graph, different letters above any two columns denote significance (p<0.05) between two columns of mean value, on the contrary, there is no significance. The component TSH1-3 has the optimal effect on improving the secretion of testosterone in the TM3 cell and thus, is selected to further study the mechanism of tonifying kidney and invigorating yang.

4. Impacts of the TSH1-3 and synthetic peptide fragment VTPVGSPR on the secretion volume of a TM3 cell-correlated male hormone

Androstenedione, testosterone, dihydrotestosterone, and free testosterone secreted by a TM3 cell were measured by an Elisa kit. The TM3 cells in the logarithmic phase were taken and paved in a 96-well plate in a density of $10^5$ CFU/mL, 100 $\mu$L per well, and cultured for 24 h in a 5% CO2, 37°C incubator; the medium was discarded, and then 100 uL of DMEM culture solution was added; the sample group (VTPVGSPR, 50, 100, and 200) was the DMEM culture solution containing 50 $\mu$g/mL, 100 $\mu$g/mL, and 200 $\mu$g/mL of TSH1-3 and containing 200 $\mu$g/mL of the synthetic peptide fragment VTPVGSPR; the cell medium in the Control contains no the above polypeptide. After continuous culture for 48 h in the incubator, cell supernatant was collected, and the amount of androstenedione, testosterone, dihydrotestosterone, and free testosterone in the supernatant was measured by an Elisa kit. The results are specifically shown in FIG. 8.

FIG. 8 shows impacts of the Fructus Trichosanthis polypeptide TSH1-3 and a synthetic peptide fragment VTPVGSPR on the secretion volume of a TM3 cell-correlated male hormone as well as the levels of ATP and mitochondrial membrane potential according to the present invention; as can be seen from FIG. 8, both the TSH1-3 and the synthetic peptide fragment VTPVGSPR may accelerate the secretion of androstenedione, testosterone, dihydrotestosterone, and free testosterone; moreover, the impact of the TSH1-3 on their secretion is dose-dependent.

Testosterone is synthesized by androstenedione under the action of 17 beta-hydroxysteroid dehydrogenase (17 $\beta$-HSD). Dihydrotestosterone (DHT) is a potent metabolite of testosterone, and is mainly subjected to $5\alpha$ reduction in specific tissues such as prostate glands, skin and liver; the local synthesis of DHT is crucial to the normal development of male characters during the prenatal and adolescence. Based on free hormone hypothesis (FHH), free testosterone can be diffused into cells and bound to androgen receptors. Testosterone bound to sex hormone-binding globulin SHBG may not be directly spread to tissues. Therefore, the free testosterone level can reflect biological activity better. The exploration on the impact of the Fructus Trichosanthis polypeptide on the secretion of the above substance is beneficial to knowing the mechanism and efficacy of tonifying kidney and invigorating yang.

5. Impacts of the TSH1-3 and the synthetic peptide fragment VTPVGSPR on the expression of proteins and genes correlated to the synthesis of testosterone in a TM3 cell

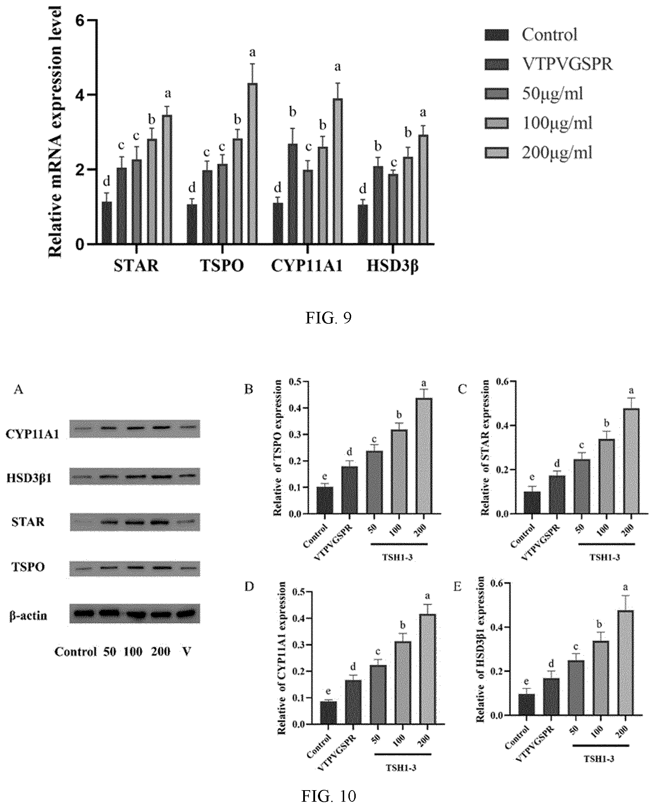

The TM3 cells ($5\times10^5$ CFU) were incubated in a 12-well plate over the night, and treated by different concentrations of TSH1-3(50, 100, 200 $\mu$g/mL) and VTPVGSPR (200 $\mu$g/mL) at 37°C for 48 h, and then a Trizol reagent was used to extract total RNA, and Control was set, and the polypeptide was replaced by the same volume of DMEM culture solution. An RNA PCR kit (CWBIO, China) was used to perform reverse transcription and PCR reaction on the extracted RNA; $\beta$-actin served as a control gene, and the relative mRNA expression level of the gene of interest was determined via a $2^{-\triangle\triangle Ct}$ method. The relative expression quantity of the gene of interest is a ratio of the experimental group to the Control, and results are shown in FIG. 9.

The TM3 cells ($5\times10^5$ CFU/well) were incubated in a 12-well plate over the night, and treated by different concentrations of TSH1-3 (50, 100, 200 $\mu$g/mL) and VTPVGSPR (200 $\mu$g/mL) at 37°C for 48 h, and the TM3 cells were collected and washed twice by PBS, then lysed with a 200 $\mu$L of radioimmunoprecipitation assay lysis buffer (RIPA), then centrifuged at 4°C and 12,000 rpm for 15 min; the obtained supernatant was the total protein extract, and the protein concentration of the lysate solution was determined by a BCA protein assay kit. The total protein extract was subjected to sodium lauryl sulfate-polyacrylamide gel electrophoresis separation with 12% SDS-PAGE gel, and the transferred onto a nitrocellulose (NC) membrane. The membrane was blocked for 1.5 h with 5% skimmed milk powder in phosphate buffered saline (PBST) containing Tween 20 at room temperature. The membrane was then incubated with a primary antibody over the night at 4°C. After being washed for three times by PBST, the membrane was incubated with a horse radish peroxidase (HRP)-labeled secondary antibody for 1.5 h at room temperature. An enhanced chemiluminiscence (ECL) detection reagent was used to detect a chemiluminescent signal, and a ChemiScope6100 imaging system (Clinx, Shanghai, China) was used to capture images, and image J software was used to analyze protein bands to obtain the expression quantity of the related proteins. The results are shown in FIG. 10.

Genes StAR, TSPO, CYP11A1, and 3$\beta$-HSD play an important role in the synthesis of testosterone; firstly, steroidogenic acute regulatory protein (StAR) is activated, and then bound to the transportprotein (TSPO) on the mitochondrial outer membrane to accelerate the transportation of cholesterol to intima from adventitia; as the raw material of synthesizing testosterone, cholesterol participates into the synthetic process of the testosterone in the TM3 cell. Afterwards, the cholesterol is transformed into pregnenolone by a cytochrome P450, a cholesterol side-chain cleavage enzyme (CYP11A1) on mitochondrial inner membrane, and pregnenolone is further transformed into endoplasmic reticulum, and then transformed into progesterone by a 3$\beta$-hydroxysteroid dehydrogenase (3$\beta$-HSD). Progesterone is then catalyzed into androstenedione, and finally transformed into testosterone. As can be seen from the results of FIGS. 9 and 10, after being interfered by the TSH1-3 and the synthetic peptide fragment VTPVGSPR, the expression level of each protein and the expression quantity of related genes are significantly improved as compared to these in the Control.

Moreover, TSH1-3 promotes the expression of StAR, TSPO, CYP11A1, and 3β-HSD in transcriptional level and protein level in a dose-dependent form, which indicates that the TSH1-3 and the synthetic peptide fragment VTPVGSPR may interfere the synthetic pathway of testosterone to promote the synthesis and secretion of testosterone.

6. Impacts of the TSH1-3 and the synthetic peptide fragment VTPVGSPR on the expression of proteins and genes correlated to the synthesis of testosterone in a TM3 cell

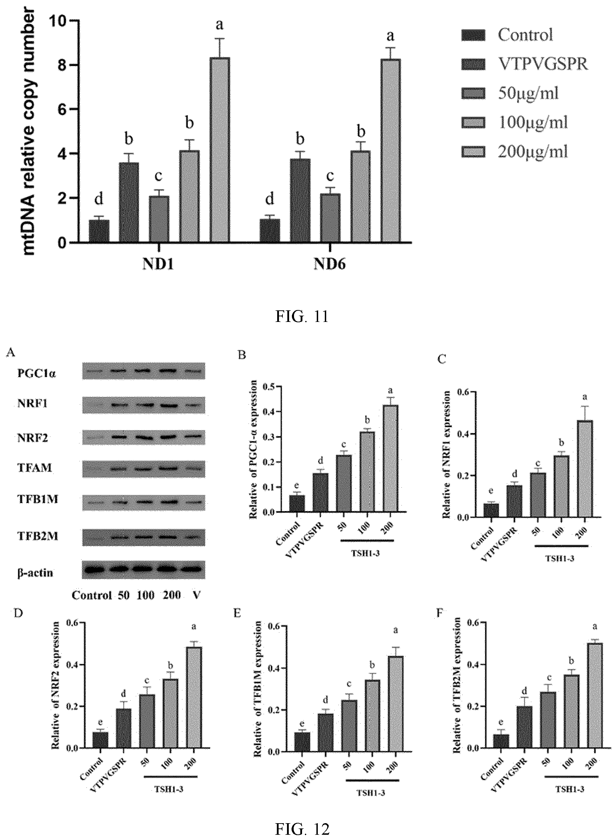

DNeasy blood and a tissue kit (Qiagen, Shanghai) were used to extract mtDNA from TM3 cells, and a specific TaqMan probe provided by Life Technologies was used to quantify the mtDNA copy number. To assess the mtDNA level, a probe directed to mitochondrial genes (ND1 and ND6) were used, and eucaryon 18S served as a normalized reference. The results are shown in FIG. 11.

The TM3 cells ($5 \times 10^5$ CFU/well) were incubated in a 12-well plate over the night, and treated by different concentrations of TSH1-3 (50, 100, 200 μg/mL) and VTPVGSPR (200 μg/mL) at 37°C for 48 h. The cells were collected and washed twice by PBS, then TM3 cells were lysed with a 200 μL of RIPA, then centrifuged at 4°C and 12,000 rpm for 15 min; the obtained supernatant was the total protein extract, and the protein concentration of the lysate solution was determined by a BCA protein assay kit. The total protein extract was subjected to sodium lauryl sulfate-polyacrylamide gel electrophoresis separation with 12% SDS-PAGE gel, and the transferred onto a nitrocellulose (NC) membrane. The membrane was blocked for 1.5 h with 5% skimmed milk powder in phosphate buffered saline (PBST) containing Tween 20 at room temperature. The membrane was then incubated with a primary antibody over the night at 4°C. After being washed for three times by PBST, the membrane was incubated with a horse radish peroxidase (HRP)-labeled secondary antibody for 1.5 h at room temperature. An enhanced chemiluminiscence (ECL) detection reagent was used to detect a chemiluminescent signal, and a ChemiScope6100 imaging system (Clinx, Shanghai, China) was used to capture images, and image J software was used to analyze protein bands to obtain the expression quantity of the related proteins. The results are shown in FIG. 12.

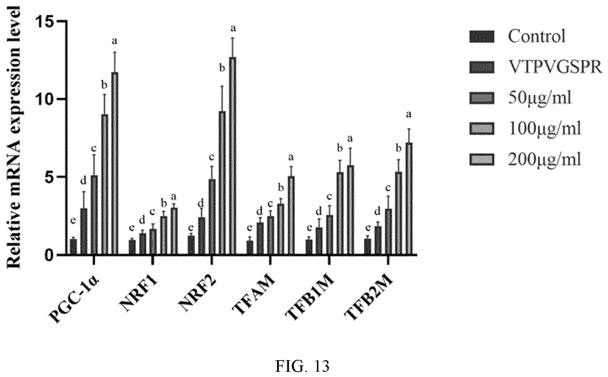

The TM3 cells ($5 \times 10^5$ CFU/well) were incubated in a 12-well plate over the night, and treated by different concentrations of TSH1-3 (50, 100, 200 μg/mL) and VTPVGSPR (200 μg/mL) at 37°C for 48 h. 1 mL Trizol reagent was used to extract total RNA from the cells, and a RNA PCR kit (CWBIO, China) was used for reverse transcription, and then a specific primers were utilized for polymerase chain reaction. B-actin served as a control gene, a $2^{-\triangle\triangle Ct}$ method was used to determine the relative mRNA expression level of the target gene; and the relative expression quantity of the target gene was calculated as a ratio of the experimental group to the Control. The results are shown in FIG. 13.

Researches show that mitochondrial biogenesis is closely correlated to the secretion of testosterone, and inhibition of secretion will lead to reduced secretion of testosterone. As shown in FIG. 11, the *Fructus Trichosanthis* polypeptide component TSH1-3 and the synthetic peptide fragment VTPVGSPR may accelerate the expression of the mitochondrial mtDNA. As shown in FIGS. 12 and 13, after being treated by the polypeptide, the genes and proteins correlated to mitochondrial biogenesis PGC-1α, TFAM, TFB1M, TFB2M, NRF1, and NRF2 have remarkably improved expression quantity. PGC-1α may coordinate the expression of the nuclear respiratory factors 1 and 2 (NRF1 and NRF2) as well as transcription factors A and B (TFAM, TFB1M, and TFB2M). TFAM, TFB1M, and TFB2M are the proteins essential to mitochondrial biogenesis. The experimental result indicates that the acceleration of the *Fructus Trichosanthis* polypeptide in the secretion of a male hormone testosterone in a TM3 cell may be achieved by modulating mitochondrial biogenesis.

In conclusion, a mouse interstitial cell TM3 served as a model in the present invention to evaluate the efficacy of the *Fructus Trichosanthis* polypeptide provided by the present invention in accelerating the secretion of testosterone. The results indicate that the *Fructus Trichosanthis* polypeptide provided in the present invention may significantly accelerate the secretion of testosterone in a TM3 cell; its mechanism of accelerating the secretion of testosterone is correlated to the expression of the related genes and proteins for modulating the secretion of testosterone as well as the expression of genes and proteins for enhancing the mitochondrial function and accelerating mitochondrial biogenesis. Therefore, the *Fructus Trichosanthis* polypeptide provided in the present invention may accelerate the secretion of testosterone in a TM3 cell, and has certain efficacy of tonifying kidney and invigorating yang, and thus may be developed as a substitute for a traditional invigorating-yang medicine.

What is described above are merely preferred embodiments of the present invention, but are not construed as limiting the present invention. Any amendment, equivalent replacement, improvement and the like made within the spirit and principle of the present invention shall fall within the protection scope of the present invention.

**Claims**

**1.** A preparation method of a *Fructus Trichosanthis* polypeptide, specifically comprising the following steps:

step 1: taking and degreasing a dehulled and crushed *Fructus Trichosanthis* seed to remove phenolic substances, and performing drying and pulverizing to obtain a *Fructus Trichosanthis* seed powder;

step 2: performing a heated water extracting to the *Fructus Trichosanthis* seed powder to obtain a *Fructus Trichosanthis* seed solution containing degenerated protein;

step 3: performing a enzymatic hydrolysis by composite enzyme to the *Fructus Trichosanthis* seed solution, after the enzymatic hydrolysis, performing enzyme deactivation and solid-liquid separation, collecting an enzymatic hydrolysate, then performing distillation under reduced pressure and drying to obtain a crude *Fructus Trichosanthis* peptide; wherein the composite enzyme comprises a neutral protease and an alkaline protease;

step 4: dissolving the crude *Fructus Trichosanthis* peptide into water to prepare a crude *Fructus Trichosanthis* peptide solution, filtering the crude *Fructus Trichosanthis* peptide solution, and performing ion-exchange resin chromatography on a filtrate obtained; serving double distilled water as an eluent, and collecting the eluent according to an appearance time of an absorbance curve, followed by concentration and drying to obtain an ion-exchange chromatography enzymolysis product, wherein a cellulose anion-exchange chromatographic column is used in the ion-exchange resin chromatography; and

step 5: dissolving the ion-exchange chromatography enzymolysis product into water, performing separation and purification by reversed-phase high-performance liquid chromatography (RP-HPLC), and collecting a protein polypeptide capable of significantly accelerating secretion of testosterone in a TM3 cell to obtain a *Fructus Trichosanthis* polypeptide; in the RP-HPLC, a chromatographic column is a C18 chromatographic column; a mobile phase A is a 0.1%-0.2% trifluoroacetic acid-aqueous solution; a mobile phase B is a 0. 1%-0.2% trifluoroacetic acid-acetonitrile solution; and gradient elution is performed by the following procedure:

for 0-3 min in a mobile phase of a 95% mobile phase A + a 5% mobile phase B;
for 3-10 min in a mobile phase of an 80% mobile phase A + a 20% mobile phase B;
for 10-20 min in a mobile phase of a 50% mobile phase A + a 50% mobile phase B;
for 20-23 min in a mobile phase of a 20% mobile phase A + an 80% mobile phase B.

2. The preparation method according to claim 1, wherein in the step 1, a degreasing solvent for the *Fructus Trichosanthis* seed is n-hexane; a weight-to-volume ratio of the *Fructus Trichosanthis* seed to the n-hexane is 1:3-6; a solvent for removing phenolic substances is acetone; a weight-to-volume ratio of the *Fructus Trichosanthis* seed to the acetone is 1 :3-6; and/or in the step 2, the heated water extracting is performed at a temperature of 90-100°C for 3-10 h, and a mass ratio of the *Fructus Trichosanthis* seed powder to water is 1:30-50.

3. The preparation method according to claim 1, wherein in the step 3, the neutral protease is a metalloprotease derived from *Bacillus subtilis;* and/or the alkaline protease is an endo protease derived from *Bacillus licheniformis.*

4. The preparation method according to claim 3, wherein an amount of the alkaline protease added accounts for 20%-40% of the *Fructus Trichosanthis* seed powder by mass; an amount of the neutral protease accounts for 20%-40% of the *Fructus Trichosanthis* seed powder by mass; and in the step 3, the composite enzymatic hydrolysis is performed at a pH of 7.5-8 or 9-9.5 and a temperature of 55-75°C for 3-5 h.

5. The preparation method according to claim 4, wherein in the step 3, the enzymatic hydrolysis is performed at a pH of 9-9.5 and a temperature of 65°C for 5 h.

6. The preparation method according to claim 1, wherein in the step 4, the eluent has a flow rate of 1-2 mL/min; and a DEAE-52 cellulose anion-exchange chromatographic column is used in the ion-exchange resin chromatography.

7. The preparation method according to claim 6, wherein in the step 4, the eluent has a flow rate of 1 mL/min, and an eluent having an appearance time of 135-250 min is collected.

8. The preparation method according to claim 1, wherein in the step 5, the chromatographic column is Pursuit XRs C-18; the mobile phase A and the mobile phase B are 0.1% trifluoroacetic acid-water and 0.1% trifluoroacetic acid-acetonitrile, respectively, and have a flow rate of 20 mL/min; an eluent having an appearance time of 10.0-12.3 min or 21.0-21.4 min is collected, to obtain a *Fructus Trichosanthis* polypeptide.

9. *KFructus Trichosanthis* polypeptide prepared by the preparation method of any one of claims 1-8.

10. Use of the *Fructus Trichosanthis* polypeptide of claim 8, comprising:

for use in manufacture of a medicament for tonifying kidney yang; or
for use in a medicament for accelerating a TM3 cell to secrete testosterone, or
for use in manufacture of a medicament for modulating expression of a gene and a protein associated with secretion of testosterone in a TM3 cell; or
for use in manufacture of a medicament for modulating expression of a gene and a protein associated with mitochondrial biogenesis in a TM3 cell.

**Amended claims in accordance with Rule 137(2) EPC.**

1. A preparation method of *Fructus Trichosanthis* peptide fragments, specifically comprising the following steps:

   step 1: taking and degreasing a dehulled and crushed *Fructus Trichosanthis* seed to remove phenolic substances, and performing drying and pulverizing to obtain a *Fructus Trichosanthis* seed powder;
   step 2: performing a heated water extracting to the *Fructus Trichosanthis* seed powder to obtain a *Fructus Trichosanthis* seed solution containing degenerated protein;
   step 3: performing a enzymatic hydrolysis by composite enzyme to the *Fructus Trichosanthis* seed solution, after the enzymatic hydrolysis, performing enzyme deactivation and solid-liquid separation, collecting an enzymatic hydrolysate, then performing distillation under reduced pressure and drying to obtain a crude *Fructus Trichosanthis* peptide; wherein the composite enzyme comprises a neutral protease and an alkaline protease;
   step 4: dissolving the crude *Fructus Trichosanthis* peptide into water to prepare a crude *Fructus Trichosanthis* peptide solution, filtering the crude *Fructus Trichosanthis* peptide solution, and performing ion-exchange resin chromatography on a filtrate obtained; serving double distilled water as an eluent, and collecting the eluent according to an appearance time of an absorbance curve, followed by concentration and drying to obtain an ion-exchange chromatography enzymolysis product, wherein a cellulose anion-exchange chromatographic column is used in the ion-exchange resin chromatography, and the eluent has a flow rate of 1 mL/min, and an eluent having an appearance time of 135-250 min is collected; and
   step 5: dissolving the ion-exchange chromatography enzymolysis product into water, performing separation and purification by reversed-phase high-performance liquid chromatography (RP-HPLC), and collecting an eluent having an appearance time of 10.0-12.3 min or 21.0-21.4 min to obtain a *Fructus Trichosanthis* peptide fragments; in the RP-HPLC, a chromatographic column is a reversed-phase high-performance liquid chromatograph (HPLC) C18 chromatographic column having a 100Å pore size, a 440 $m^2$/g surface area and 22% carbon load; a mobile phase A is a 0.1% trifluoroacetic acid-aqueous solution; a mobile phase B is a 0.1% trifluoroacetic acid-acetonitrile solution and have a flow rate of 20 mL/min; and gradient elution is performed by the following procedure:

   for 0-3 min in a mobile phase of a 95% mobile phase A + a 5% mobile phase B;
   for 3-10 min in a mobile phase of an 80% mobile phase A + a 20% mobile phase B;
   for 10-20 min in a mobile phase of a 50% mobile phase A + a 50% mobile phase B;
   for 20-23 min in a mobile phase of a 20% mobile phase A + an 80% mobile phase B.

2. The preparation method according to claim 1, wherein in the step 1, a degreasing solvent for the *Fructus Trichosanthis* seed is n-hexane; a weight-to-volume ratio of the *Fructus Trichosanthis* seed to the n-hexane is 1:3-6; a solvent for removing phenolic substances is acetone; a weight-to-volume ratio of the *Fructus Trichosanthis* seed to the acetone is 1:3-6; and/or in the step 2, the heated water extracting is performed at a temperature of 90-100°C for 3-10 h, and a mass ratio of the *Fructus Trichosanthis* seed powder to water is 1:30-50.

3. The preparation method according to claim 1, wherein in the step 3, the neutral protease is a metalloprotease derived from *Bacillus subtilis;* and/or the alkaline protease is an endo protease derived from *Bacillus licheniformis.*

4. The preparation method according to claim 3, wherein an amount of the alkaline protease added accounts for 20%-40% of the *Fructus Trichosanthis* seed powder by mass; an amount of the neutral protease accounts for 20%-40% of the *Fructus Trichosanthis* seed powder by mass; and in the step 3, the composite enzymatic hydrolysis is performed at a pH of 7.5-8 or 9-9.5 and a temperature of 55-75°C for 3-5 h.

5. The preparation method according to claim 4, wherein in the step 3, the enzymatic hydrolysis is performed at a pH of 9-9.5 and a temperature of 65°C for 5 h.

6. The preparation method according to claim 1, wherein in the step 4, the eluent has a flow rate of 1-2 mL/min; and a DEAE-52 cellulose anion-exchange chromatographic column is used in the ion-exchange resin chromatography.

7. A *Fructus Trichosanthis* peptide fragment composition prepared by the preparation method of any one of claims 1-8.

8. A *Fructus Trichosanthis* peptide fragment composition prepared by the method of claim 1 for use in the treatment of testosterone deficiency.

FIG. 1

FIG. 2

FIG. 3

FIG. 4

FIG. 5

FIG. 6

FIG. 7

FIG. 8

FIG. 9

FIG. 10

FIG. 11

FIG. 12

FIG. 13

Europäisches
Patentamt

European
Patent Office

Office européen
des brevets

# EUROPEAN SEARCH REPORT

**Application Number**

EP 24 22 3872

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X,P | HE ZHILIN ET AL: "Testosterone-promoting effects of Trichosanthes kirilowii-derived peptides on TM3 cells", FOOD BIOSCIENCE, vol. 61, 6 August 2024 (2024-08-06), page 104890, XP093260210, NL ISSN: 2212-4292, DOI: 10.1016/j.fbio.2024.104890 * Abstract; page 2, 2.1, 2.2, 2.5; pages 8-11, 3.2-3.7; figures 1-2, 4-7; table 3 * | 1-10 | INV. A61K36/428 A61K38/01 A61K38/04 A61P5/26 A61P13/12 C07K7/00 C12P21/06 A01H5/10 A01H6/34 C07K14/415 |
| X | CN 113 769 062 B (NANJING UNIVERSITY OF TECHNOLOGY) 20 June 2023 (2023-06-20) * example 1 * | 9,10 | |
| X | US 10 905 107 B2 (KOREA ADVANCED INST SCI & TECH [KR]) 2 February 2021 (2021-02-02) * column 3 - column 3 * | 9 | |
| A | CN 112 656 832 A (HANGZHOU WAHAHA TECHNOLOGY CO LTD) 16 April 2021 (2021-04-16) * claims 3, 4, 9, 10; example 2; table 1 * | 1-10 | TECHNICAL FIELDS SEARCHED (IPC) A61K C07K C12P A61P |

-/--

The present search report has been drawn up for all claims

| Place of search | Date of completion of the search | Examiner |
|---|---|---|
| Munich | 6 May 2025 | Basso, Veronica |

EPO FORM 1503 03.82 (P04C01)

page 1 of 2

| | Europäisches Patentamt European Patent Office Office européen des brevets | **EUROPEAN SEARCH REPORT** | Application Number EP 24 22 3872 |
|---|---|---|---|

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | NG T B ET AL: "Effects of @a-momorcharin, @b-momorcharin and @a-trichosanthin on lipogenesis and testicular and adrenal steroidogenesis in vitro and plasma-glucose levels in vivo", JOURNAL OF ETHNOPHARMACOLOGY, ELSEVIER IRELAND LTD, IE, vol. 18, no. 1, 1 October 1986 (1986-10-01), pages 45-53, XP023802974, ISSN: 0378-8741, DOI: 10.1016/0378-8741(86)90042-5 [retrieved on 1986-10-01] * Abstract; page 49; table 2 * ----- | 1-10 | |
| A | SEO CHANG-SEOB ET AL: "Trichosanthes kirilowii ameliorates cisplatin-induced nephrotoxicity in both in vitro and in vivo", NATURAL PRODUCT RESEARCH, vol. 29, no. 6, 4 September 2014 (2014-09-04), pages 554-557, XP093260303, GB ISSN: 1478-6419, DOI: 10.1080/14786419.2014.952229 * the whole document * ----- | 1-10 | TECHNICAL FIELDS SEARCHED (IPC) |

The present search report has been drawn up for all claims

| Place of search | Date of completion of the search | Examiner |
|---|---|---|
| Munich | 6 May 2025 | Basso, Veronica |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
................................................................
& : member of the same patent family, corresponding document

EPO FORM 1503 03.82 (P04C01)

page 2 of 2

## ANNEX TO THE EUROPEAN SEARCH REPORT
## ON EUROPEAN PATENT APPLICATION NO.

EP 24 22 3872

This annex lists the patent family members relating to the patent documents cited in the above-mentioned European search report.
The members are as contained in the European Patent Office EDP file on
The European Patent Office is in no way liable for these particulars which are merely given for the purpose of information.

06-05-2025

| Patent document cited in search report | | Publication date | Patent family member(s) | | Publication date |
|---|---|---|---|---|---|
| CN 113769062 | B | 20-06-2023 | NONE | | |
| US 10905107 | B2 | 02-02-2021 | KR 20180027383 A | | 14-03-2018 |
| | | | US 2018064076 A1 | | 08-03-2018 |
| CN 112656832 | A | 16-04-2021 | NONE | | |

EPO FORM P0459

For more details about this annex : see Official Journal of the European Patent Office, No. 12/82